# EUROPÄISCHE PATENTANMELDUNG

(11) **EP 2 133 067 A1**
(43) Veröffentlichungstag der Anmeldung: **16.12.2009**
(21) Anmeldenummer: 09005804.1
(22) Anmeldetag: 27.04.2009
(51) Int. Cl.: A61K 8/92, A61Q 1/02, A61Q 1/06, A61Q 1/10, A61K 8/19, A61K 8/29, A61K 8/46, A61K 8/26

(54) **Kosmetische Zubereitung enthaltend Sonnenblumenwachs**

(30) Priorität: 14.05.2008 DE 102008001776
(71) Anmelder: Beiersdorf AG, 20253 Hamburg (DE)
(72) Erfinder: Münchow, Lynn, 22547 Hamburg (DE); Scheede, Stefan, 20255 Hamburg (DE); Zoltowski, Craig, Norwalk CT 06854 (US)

(57) **Zusammenfassung**

Kosmetische Zubereitung enthaltend
a) Sonnenblumenwachs,
b) ein oder mehrere Pigmente.

## Beschreibung

Die vorliegende Erfindung betrifft eine kosmetische Zubereitung enthaltend Sonnenblumenwachs und Pigmente.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.
Das Gesichts-Make-up soll der Gesichtshaut ein natürliches Aussehen verleihen, blasse Haut auffrischen, farbliche Unregelmäßigkeiten der Haut ausgleichen sowie Augen und Lippen besonders betonen.

Neben Gesichtspudern und Rouge als pulverförmigen Kosmetika sowie stiftförmigen Zubereitungen werden hierzu cremeförmige Präparate, wie Tagescremes und Creme-Make-up auf Emulsionsbasis verwendet.

Dekorative Kosmetika enthalten Farbstoffe, meist in Form von Pigmenten. An derartige Zubereitungen wird die Anforderung gestellt, einerseits möglichst ortsfest auf der Haut oder den Wimpern zu verbleiben und nicht unkontrolliert beispielsweise durch Schweiß oder Tränen zu verschmieren oder, bei direktem Kontakt mit anderen Oberflächen, auf diese zu transferieren. Andererseits sollte sich die Zubereitung problemlos direkt (z.B. Lippenstift) oder mit Hilfe eines Applikators (z.B. Mascarabürste) auf die menschliche Oberfläche (Haut, Haar, Wimper) auftragen und auf Wunsch auch problemlos wieder entfernen lassen.

Um diesem Anforderungsprofil möglichst nahe zu kommen bedarf es hoch komplexer, ausgeklügelter Substanzmischungen.

Es war daher die Aufgabe der vorliegenden Erfindung, eine dekorative kosmetische Zusammensetzung zu entwickeln, welche das beschriebene Anforderungsprofil besonders gut erfüllt.

Darüber hinaus sollten die Zubereitungen besonders einfach und kostengünstig herstellbar sein.

Eine wachsende Zahl an Verbrauchern verlangt von den verwendeten Kosmetika, dass deren Inhaltsstoffe möglichst.natürlichen Ursprungs sind und deren Herstellung und Entsorgung die Umwelt möglichst wenig belastet.

Es war daher die Aufgabe der vorliegenden Erfindung, ein dekoratives Kosmetikum zu entwickeln, das diesen Anforderungen nachkommt.

Überraschend gelöst werden die Aufgaben durch eine kosmetische Zubereitung enthaltend
a) Sonnenblumenwachs,
b) ein oder mehrere Pigmente.

Die erfindungsgemäßen Zubereitungen werden erfindungsgemäß vorteilhaft als Lippenstift (Farbstift (bevorzugt) oder "lip gloss"), Lidschatten ("eye shadow"), Mascara, Foundation ("foundation", "blush"), Kajal-Zubereitung ("eye liner") eingesetzt. Erfindungsgemäß bevorzugt sind dabei Lippenstifte und Mascara, wobei die Mascara erfindungsgemäß besonders bevorzugt ist.

Die erfindungsgemäßen Zubereitungen führen bei der Anwendung, insbesondere, wenn sie als Mascara vorliegen, zu Zubereitungen, die ein hervorragendes (Wimpern-) Volumen, einen glatten, nicht brüchigen Film, eine hohe Wasserresistenz , Weichheit und Elastizität aufweisen. Die Zubereitungen lassen sich besonders leicht und gleichmäßig applizieren.

Einen besonderen Vorteil der erfindungsgemäßen Zubereitungen stellen deren Transfereigenschaften dar. Die Zubereitungen lassen sich zunächst einfach und bequem applizieren und weisen nach der Applikation eine hohe Transferresistenz auf (siehe Vergleichsversuche).

Erfindungsgemäß vorteilhafte Ausführungsformen der vorliegenden Erfindung sind **dadurch gekennzeichnet, dass** die Zubereitung einen Transferwert von weniger als 55000 aufweist.

Erfindungsgemäß bevorzugte Ausführungsformen der vorliegenden Erfindung sind **dadurch gekennzeichnet, dass** die Zubereitung einen Transferwert von weniger als 20000 aufweist.

Erfindungsgemäß besonders bevorzugte Ausführungsformen der vorliegenden Erfindung sind **dadurch gekennzeichnet, dass** die Zubereitung einen Transferwert von weniger als 1000 aufweist.

Erfindungsgemäß besonders bevorzugte Ausführungsformen der vorliegenden Erfindung sind **dadurch gekennzeichnet, dass** die Zubereitung einen Transferwert von weniger als 500 aufweist.

Der erfindungsgemäße Transferwert wird dabei mit dem folgenden Transfer Test gemessen:

**Transfer Test**

| | |
|---|---|
| 1. Prinzip: | Der Transfer wird durch den Abklatsch (Abdruck) eines Mascarafilms auf ein Papier simuliert. Je stärker der Abdruck auf dem Papier ist, umso stärker ist der Transfer-Effekt und der Transferwert. |
| | |
| 2. Material: | Pastellpapier für Pastell-und Ölpastellkreiden (*Säure-frei, alterungsbeständig, 25% Hadern. Beispiel Grösse 17 x 24 cm. Ref. No. 10628950. Hahnemühle Fine Arts, 170 g*/*m², mittelrauh).* Großverbraucher-Küchenpapier (*Profix multi. 750 Tücher a 40 x 38 cm. Temca GmbH. Artikelnummer 005150).* Schablone für Mascarafilmauftrag (*Dicke 0,3 mm. Durchmesser 20mm*) Rolle mit Griff (*Rad-Breite= 4,6cm. Rad-Durchmesser= 6 cm. Rad Gewicht= 1 KG. Insgesamt 1,17 kg mit Griff).* Pipette VES-Wasser (25 °C) Stoppuhr |
| | |
| 3. Methode: | Das Küchenpapier wird in 5 cm x 5 cm große Stücke geschnitten. Mit Hilfe der Schablone wird ein kreisrunder Film der zu testenden Mascara auf das Pastellkreidenpapier auftragen und mit einem Objektträger die Oberfläche glatt gestrichen. Dadurch wird eine definierte Menge Mascara in einer definierten Filmdicke auf dem Pastellkreidepapier erzeugt. Der Film wird über Nacht bei Raumtemperatur trocknen gelassen. |
| | Am nächsten Tag wird mit der Pipette (2 mL Größe) 10 Tropfen VE-Wasser (Raumtemperatur) auf den getrockneten Mascarafilm auftragen (gleichmäßig) und 5 Minuten einwirken gelassen. |
| | |
| | Nach 5 Minuten wird der befeuchtete Mascarafilm mit dem Küchenpapier (5 cm x 5 cm Größe) vorsichtig abgedeckt. Anschließend wird die Rolle gleichmäßig in 15 Sekunden 15- mal über den mit dem Küchenpapier abgedeckten Film gerollt. Um eine reproduzierbare Gewichtsbelastung zu erzeugen, wird die Rolle so bewegt, dass der Druck auf die Probe ausschließlich durch das Eigengewicht der Rolle erzeugt wird. Durch diese Behandlung wird ein Abdruck der Mascara auf dem Küchenpapier erzeugt. Anschließend wird das Küchenpapier vom Mascarafilm vorsichtig entfernt und bei Raumtemperatur getrocknet. |
| | |
| | Um aussagekräftige Ergebnisse zu erhalten, wird eine Messreihe aus mindestens 5 Versuchen unter gleichen Bedingungen durchgeführt. |

Um zu den erfindungsgemäßen Transferwerten zu gelangen, werden die Abdrücke wie folgt ausgewertet:
Das mit dem Abdruck versehene Kuchenpapier wird nach dem Trocknen über einen Scanner eingescannt (Tiff.-Format, 800 dpi, Schwarz/Weiß-Format mit Schwarz/Weiß-Skala). Das Papier sollte dabei auf einem weißen Hintergrund eingescannt werden. Als Referenz wird ein entsprechendes Küchenpapier ohne Abdruck ebenfalls eingescannt. Die Referenz dient u.a. zur Bestimmung des Farbbereiches, in dem das Küchenpapier seine Eigenfarbe hat.
   Die Schwarz/ Weiss-Skala hat eine "L" Skala (wo Schwarz = 0 und Weiss=255) von 0 bis 255.
Es wird nun die Fläche der schwarzen Pixeln- d.h. die Summe der Pixeln die auf der "L"-Skala einen Wert von 0 bis zu 226 aufweisen, bestimmt (im Bereich über 226 liegen die Farbwerte des Küchenpapiers). Von dieser Summe werden die Summe der Pixeln im Bereich von 0 bis 226 auf der L-Skala des Küchenpapiers ohne Abdruck abgezogen.

Die Auswertung erfolgt über ein Computer-Programm wie Photoshop oder Image J. Wichtig ist natürlich nur das Bereich zu messen wo man den Transfer Test gemacht hat ohne Ränder und Hintergrund usw.

Es ist erfindungsgemäß vorteilhaft, wenn das Sonnenblumenwachs gesättigte Ester mit einer Kohlenstoffanzahl von 42 bis 60 enthält, die gebildet werden aus C₂₀ bis C₃₂- Fettalkoholen und C₂₀ bis C₂₈-Fettsäuren.

Es ist erfindungsgemäß bevorzugt, wenn als Sonnenblumenwachs die Verbindung CAS 93028-82-1 eingesetzt wird.

Die erfindungsgemäße Zubereitung kann neben Sonnenblumenwachs weitere Wachse enthalten. Diese weitere Wachse werden erfindungsgemäß vorteilhaft gewählt aus der Gruppe der folgenden Wachse: Bienenwachs, Paraffinwachs, Carnaubawachs, Candellilawachs, Schellackwachs.

Es ist erfindungsgemäß vorteilhaft, wenn die erfindungsgemäße Zubereitung Wachse in einer Gesamtmenge von 3,5 bis 50 Gewichts-%, bezogen auf das Gesamtgewicht der Zubereitung, enthält.

Es ist erfindungsgemäß bevorzugt, wenn die erfindungsgemäße Zubereitung Wachse in einer Gesamtmenge von 8 bis 20 Gewichts-%, bezogen auf das Gesamtgewicht der Zubereitung, enthält.

Erfindungsgemäß vorteilhaft beträgt der Gehalt an Sonnenblumenwachs in der Zubereitung von 0,5 bis 10 Gewichts-%, bezogen auf das Gesamtgewicht der Zubereitung.

Erfindungsgemäß bevorzugt beträgt der Gehalt an Sonnenblumenwachs in der Zubereitung von 1 bis 3 Gewichts-%, bezogen auf das Gesamtgewicht der Zubereitung.

Es ist erfindungsgemäß vorteilhaft, wenn ein oder mehrere Pigmente gewählt werden aus der Gruppe der Verbindungen Schwarzes Eisenoxid, Kohlenstoff (Carbon black =Cl 77266, D&C Black 2) und/oder Stoffe mit dem Color Index Cl 77891, 75470, 17200, 77891, 77007, 77499,77491,77492.

Dabei ist der Einsatz von schwarzem Eisenoxid erfindungsgemäß bevorzugt.

Es ist erfindungsgemäß vorteilhaft, wenn die erfindungsgemäße Zubereitung Pigmente in einer Gesamtmenge von 2 bis 15 Gewichts-%, bezogen auf das Gesamtgewicht der Zubereitung, enthält.

Es ist erfindungsgemäß bevorzugt, wenn die erfindungsgemäße Zubereitung Pigmente in einer Gesamtmenge von 4 bis 10 Gewichts-%, bezogen auf das Gesamtgewicht der Zubereitung, enthält.

Es ist erfindungsgemäß vorteilhaft, wenn die erfindungsgemäße Zubereitung ein oder mehrere Organosiloxane Verbindungen enthält. Es ist in diesem Falle erfindungsgemäß bevorzugt, wenn die Zubereitung Trimethylsiloxysilikat als Organosiloxan enthält. In einer erfindungsgemäß besonderen Ausführungsform enthält die Zubereitung ausschließlich Trimethylsiloxysilikat als Organosiloxan.

Es ist erfindungsgemäß vorteilhaft, wenn die erfindungsgemäße Zubereitung Organosiloxane in einer Gesamtmenge von 1 bis 10 Gewichts-%, bezogen auf das Gesamtgewicht der Zubereitung, enthält.

Es ist erfindungsgemäß bevorzugt, wenn die erfindungsgemäße Zubereitung Organosiloxane in einer Gesamtmenge von 2 bis 6 Gewichts-%, bezogen auf das Gesamtgewicht der Zubereitung, enthält.

Es ist erfindungsgemäß vorteilhaft, wenn die erfindungsgemäße Zubereitung ein oder mehrere oberflächenaktive Verbindungen enthält. Es ist in diesem Fall erfindungsgemäß bevorzugt, wenn die Zubereitung Polyglyceryl-3 Diisostearat als oberflächenaktive Verbindung enthält. In einer erfindungsgemäß besonderen Ausführungsform enthält die Zubereitung ausschließlich Polyglyceryl-3 Diisostearat als oberflächenaktive Verbindung.

Es ist erfindungsgemäß vorteilhaft, wenn die erfindungsgemäße Zubereitung oberflächenaktive Verbindungen in einer Gesamtmenge von 0,1 bis 4 Gewichts-%, bezogen auf das Gesamtgewicht der Zubereitung, enthält.

Es ist erfindungsgemäß bevorzugt, wenn die erfindungsgemäße Zubereitung oberflächenaktive in einer Gesamtmenge von 0,2 bis 1,5 Gewichts-%, bezogen auf das Gesamtgewicht der Zubereitung, enthält.

Es ist erfindungsgemäß vorteilhaft, wenn die erfindungsgemäße Zubereitung wasserfrei ist. "Wasserfrei" bedeutet im Rahmen der vorliegenden Erfindung, dass der Zubereitung bei der Herstellung kein Wasser zugesetzt wird und die Zubereitung, außer den gegebenenfalls an einzelnen Rohstoffen durch die Lagerung/Herstellung anhaftenden Wasserspuren, kein Wasser enthält.

Fakultativ kann die erfindungsgemäße Zubereitung auch weitere, für den jeweiligen Produkt-Typ typische Inhaltstoffe enthalten.

Die Ölphase kann beispielsweise vorteilhaft Verbindungen aus der Gruppe der polaren Öle enthalten, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*) und/oder Diethylhexylnaphthalat (*Hallbrite TQ oder Corapan TQ von Symrise)*.

Auch beliebige Abmischungen solcher Komponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
wobei die verwendeten Mengenateile so gewählt werden, dass die Menge des Wasserstoffes des Organopolysiloxans (1) oder der ungesättigten aliphatischen Gruppen des Organopolysiloxans (2)
- im Bereich von 1 bis 20 mol-% liegt, wenn das Organopolysiloxan nicht zyklisch ist und
- im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Verdickungsmittel, anfeuchtende und/oder feuchhaltende Substanzen, Füllstoffe, die das Hautgefühl verbessern, UV-Filter oder andere übliche Bestandteile einer kosmetischen oder dermatologischen Formulierung wie Alkohole, Polyole, Polymere.

Erfindungsgemäß vorteilhaft ist es, wenn die erfindungsgemäße Zubereitung als Mascara-Zubereitung verwendet wird. Wird die erfindungsgemäße Zubereitung als Mascara eingesetzt, so ist es erfindungsgemäß besonders vorteilhaft, wenn die Zubereitung mit Hilfe einer Bürste aus thermoplastischem Polyurethan aufgetragen wird. Daher ist auch ein Mascara-Produkt aus einer Bürste aus thermoplastischem Polyurethan und der erfindungsgemäßen Zubereitung erfindungsgemäß.

### Vergleichsversuche

Die folgenden Vergleichsversuche können die vorliegende Erfindung verdeutlichen:

| | **Z_WP_65** | **Z_WP_68** |
|---|---|---|
| **INCI-Name(n)** | **m [%]** | **m [%]** |
| BHT | 0,0500 | 0,0500 |
| Disteardimonium Hectorite | 6,0000 | 6,0000 |
| Helianthus Annuus (Sunflower) Seed Wax | 17,0000 | |
| Hydrogenated Polydecene | 0,5000 | 17,5000 |
| Iron Oxides | 9,0000 | 9,0000 |
| Isododecane | 51,7500 | 51,7500 |
| Polyethylene | 7,0000 | 7,0000 |
| Polyglyceryl-3 Diisostearate | 1,5000 | 1,5000 |
| Propylene Carbonate | 3,0000 | 3,0000 |
| Propylparaben | 0,2000 | 0,2000 |
| Tocopheryl Acetate | 1,0000 | 1,0000 |
| Trimethylsiloxysilicate | 3,0000 | 3,0000 |
| **Summe** | **100,0000** | **100,0000** |
| **Transferwert** | **1426** | **691826** |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Lippenstifte

| **Inhaltsstoff (INCI-Bezeichnung)** | **Anteil in Gew.-%** | | |
|---|---|---|---|
| | **1** | **2** | **3** |
| Rizinusöl | ad 100 | | 6 |
| Cocoglycerides | | ad 100 | |
| Caprylic-/Capric Triglycerides | 10 | 20 | ad 100 |
| Pentaerythrityl Tetraisistearate | | 5 | |
| Macadamiaöl | | | 2 |
| Octyldodecanol | 20 | 5 | 6 |
| Hydrogenated Polyisobuten | | | 10 |
| (z.B. Parleam Type 4, Rossow Cosmétiques) | | | |
| Polyisobutene | | 2 | 3 |
| Isopropylpalmitat | | 3,5 | 6 |
| Jojobaöl | 6 | | |
| Lanolin Öl | 5 | | 9 |
| Polyglyceryl-3 Diisostearat | 3,7 | 3,5 | |
| Bis-Diglyceryl Polyacyladipat-2 | | 6 | |
| Cetearyl Alkohol | 6 | 0,5 | |
| Cetylpalmitat | 7,5 | | |
| C20-40 Alkyl Stearat | 16 | | 3 |
| Carnaubawachs | | 5 | 4 |
| Candelillawachs | | 3 | 4 |
| Sonnenblumenwachs | 6 | 4 | 3 |
| Mikrokristallines Wachs | | 8 | 4 |
| PVP / Eicosen Copolymer | | | 0,5 |
| 4-Methylbenzyliden Campher | | | |
| Octyl Methoxycinnamat | 3 | | |
| Lauroyl Lysin | 0,5 | | 0,6 |
| Red 7 Lake | 0,5 | 2,6 | 4,2 |
| Titandioxid | | 0,6 | 1 |
| Eisenoxide | | 2,4 | 2 |
| Effektpigmente | | 6 | 3,5 |
| Glimmer | | | 4 |
| Tocopheryl Acetate | 0,5 | | 0,5 |
| Wasser | 2,5 | | |
| Parfum, Konservierungsmittel, BHT, Neutralisationsmittel, Sequestriermittel | q. s. | q. s. | q. s. |

### Lippenstifte

| | **1** | **2** | **3** | **4** |
|---|---|---|---|---|
| Synthetic Wax | 11 | 10 | 7 | 4 |
| Schellackwachs | | | | 2 |
| Sonnenblumenwachs | 3,5 | 4 | 5 | 8 |
| Isododecan | 50 | 40 | 60 | 40 |
| Pigmente | 10 | 10 | 10 | 10 |
| Trimethylsiloxysilicat | 10 | 8 | 5 | 5 |
| Sucroseacetat Ester | | 2 | | 2 |
| PVP/Hexadecene Copolymer | 2 | | | |
| Polyglyceryl-3 Diisostearat | | | | 1 |
| PVP/Eicosene Copolymer | | | 1 | |
| Propylparaben | 0,1 | 0,1 | 0,1 | 0,1 |
| BHT | 0,03 | 0,03 | 0,03 | 0,03 |
| Polyisobuten | ad 100 | | | |
| Hydrogenated Polydecene | | ad 100 | ad 100 | ad 100 |

### W/O-Foundation

| inci | Gew.% |
|---|---|
| Cl 77891 | 9 |
| Cl 77491 | 0,168 |
| Cl 77492 | 2,35 |
| Cl 77007 | 0,0168 |
| Cl 77163 | 3 |
| Aqua | ad 100 |
| Glycerin | 12 |
| Caprylic/Capric Triglyceride | 5 |
| Octyldodecanol | 5 |
| Dicaprylyl Carbonate | 6 |
| Simethicone | 0,5 |
| Polyglyceryl-3 Diisostearate | 3 |
| Sonnenblumenwachs | 4 |
| Polyethylen Wachs | 6 |
| Methyl Methacrylate Crosspolymer | 3 |
| Konservierungsmittel, Parfum | q.s. |

### W/S-Foundations:

| | **1** | **2** | **3** | **4** | **5** |
|---|---|---|---|---|---|
| | Gew.-% | Gew.-% | Gew.-% | Gew.-% | Gew.-% |
| Wasser | Ad 100 | Ad 100 | Ad 100 | Ad 100 | Ad 100 |
| Glycerin | 7 | 5 | 5 | 10 | 10 |
| Cyclomethicon + PEG/PPG-19/19 Dimethicon | | 4 | 2 | | |
| Cetyl PEG/PPG-10/1 Dimethicon | 4 | | | 5 | 5 |
| Propylene Carbonat | 0,05 | 0,06 | 0,06 | 0,03 | 0,03 |
| Disteardimonium Hectorite | 0,25 | 0,4 | 0,2 | 0,1 | 0,1 |
| Sodium Chloride | 2 | | 1 | 2 | 2 |
| Squalan | 0,5 | | 0,5 | | |
| Dicaprylyl Carbonate | | 5 | | 5 | 5 |
| Cyclomethicone | 10 | 12 | 20 | 15 | 15 |
| Dicaprylyl Ether | 2 | | | | |
| Caprylic/Capric Triglycerid | 2 | | | | |
| Dimethicon | 2 | 3 | | 4 | 4 |
| Sonnenblumenwachs | 1,5 | 4 | 3 | 3,5 | 3,5 |
| Myristyl Laktat | | | 1 | | |
| Lecithin | 1 | | 0,5 | | |
| Dimethicon + Trimethylsiloxysilikat | | | 3,5 | 2 | 2 |
| Lauroyl Lysine | 5 | | 2,5 | 3 | 3 |
| Talkum | 2 | 3 | 0,5 | | |
| Nylon 6/12 | | 3 | 2 | 4 | 4 |
| Silica | | 3 | 4 | 3 | 3 |
| Dimethicon + Dimethicon Crosspolymer | 6 | 4 | | | |
| Methyl Methacrylat Crosspolymer | | | 1 | 2 | 2 |
| Polymethylsilsesquioxan | 2 | 1 | | | |
| VP/VA Copolymer | 0,1 | | | 0,2 | 0,2 |
| VP/Hexadecen Copolymer | 0,1 | | 0,5 | | |
| Titandioxid (Cl 77891) | 6 | 3 | 3 | 8 | 8 |
| Farbpigmente (Cl 77492 + Cl 77491 + Cl 77499) | 5 | 5 | 2 | 7 | 7 |
| Effektpigmente (z.B. beschichtetes Mica) | | 3 | 1 | | |
| Titandioxid | 1 | 2 | | | |
| Dimethicodiethylbenzalmalonat (Polysilicone-15) | 0,5 | 2 | 4 | 2,5 | 2,5 |
| 2-Phenylbenzimidazol-5-sulfonsäuresalze | 1 | 5 | 2 | 3 | 3 |
| Polyglyceryl-3 Diisostearat | | | | | 1 |
| Sodium Ascorbyl Phosphate | | | 0,1 | 0,2 | 0,2 |
| Tocopheryl Acetate | 0,5 | 0,5 | 1 | 1,5 | 1,5 |
| Ubiquinon, Q10 | | 0,05 | | | |
| Phenoxyethanol + Parabene | 1 | 0,7 | | | |
| Tetrasodium Iminodisuccinat | | 0,2 | | | |
| Diazolidinyl Urea | | | 0,3 | 0,3 | 0,3 |
| Parfum | 0,5 | 0,5 | 0,5 | 0,5 | 0,5 |

## Patentansprüche

1. Kosmetische Zubereitung enthaltend
a) Sonnenblumenwachs,
b) ein oder mehrere Pigmente.

2. Kosmetische Zubereitung nach Anspruch 1, **dadurch gekennzeichnet, dass** die Zubereitung einen Transferwert von weniger als 55000 aufweist.

3. Kosmetische Zubereitung nach einem der vorhergehenden Ansprüche, **dadurch gekennzeichnet, dass** das Sonnenblumenwachs gesättigte Ester mit einer Kohlenstoffanzahl von 42 bis 60 enthält, die gebildet werden aus C20 bis C32-Fettalkoholen und C20 bis C28 Fettsäuren.

4. Kosmetische Zubereitung nach einem der vorhergehenden Ansprüche, **dadurch gekennzeichnet, dass** als Sonnenblumenwachs die Verbindung CAS 93028-82-1 eingesetzt wird.

5. Kosmetische Zubereitung nach einem der vorhergehenden Ansprüche**, dadurch gekennzeichnet, dass** die Zubereitung Wachse in einer Gesamtmenge von 3,5 bis 50 Gewichts-%, bezogen auf das Gesamtgewicht der Zubereitung, enthalten.

6. Kosmetische Zubereitung nach einem der vorhergehenden Ansprüche, **dadurch gekennzeichnet, dass** ein oder mehrere Pigmente gewählt werden aus der Gruppe der Verbindungen schwarzem Eisenoxid, Kohlenstoff und/oder Stoffe mit dem Color Index Cl 77891, 75470, 17200, 77891, 77007, 77499, 77491, 77492.

7. Kosmetische Zubereitung nach einem der vorhergehenden Ansprüche, **dadurch gekennzeichnet, dass** die Zubereitung Pigmente in einer Gesamtmenge von bis Gewichts-%, bezogen auf das Gesamtgewicht der Zubereitung, enthält.

8. Kosmetische Zubereitung nach einem der vorhergehenden Ansprüche, **dadurch gekennzeichnet, dass** die Zubereitung Trimethylsiloxysilikat enthält.

9. Kosmetische Zubereitung nach einem der vorhergehenden Ansprüche, **dadurch gekennzeichnet, dass** die Zubereitung Polyglyceryl-3 Diisostearat enthält.

10. Kosmetische Zubereitung nach einem der vorhergehenden Ansprüche, **dadurch gekennzeichnet, dass** die Zubereitung wasserfrei ist.

11. Verwendung einer kosmetischen Zubereitung nach einem der vorhergehenden Ansprüche als Lippenstift, Lidschatten, Mascara, Foundation, Kajal-Zubereitung.

12. Mascara-Produkt aus
a) einer Zubereitung nach einem der Ansprüche 1 bis 10 und
b) einer Mascara-Bürste aus thermoplastischem Polyurethan.
